(19) Europäisches Patentamt
European Patent Office
Office européen des brevets

(11) **EP 2 989 970 A1**

(12) **EUROPEAN PATENT APPLICATION**

(43) Date of publication:
**02.03.2016 Bulletin 2016/09**

(51) Int Cl.:
***A61B 5/00*** *(2006.01)*

(21) Application number: **15180843.3**

(22) Date of filing: **13.08.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **27.08.2014 JP 2014172525**
**10.06.2015 JP 2015117149**

(71) Applicant: **PreXion Corporation
Chiyoda-ku
Tokyo 101-0041 (JP)**

(72) Inventors:
• **NAKATSUKA, Hitoshi
Chiyoda-ku, Tokyo 101-0041 (JP)**
• **AGANO, Toshitaka
Chiyoda-ku, Tokyo 101-0041 (JP)**

(74) Representative: **Grünecker Patent- und
Rechtsanwälte
PartG mbB
Leopoldstraße 4
80802 München (DE)**

(54) **PHOTOACOUSTIC IMAGER**

(57)  This photoacoustic imager (100, 200, 300, 400, 500, 600) includes a light source portion (11, 211, 511, 611) and a detection portion(12), and is configured to set pulsed light emitted by the light source portion to a pulse width tw expressed in the following expression, assuming that fmax represents the maximum frequency of an acoustic wave $\underline{A}$ detected by the detection portion:

$$0.5/fmax \le tw \le 1/fmax$$

FIG.1

**Description**

BACKGROUND OF THE INVENTION

Field of the Invention

The present invention relates to a photoacoustic imager, and more particularly, it relates to a photoacoustic imager including a detection portion detecting an acoustic wave resulting from light applied to a specimen and an ultrasonic wave applied to and reflected by the specimen.

Description of the Background Art

A photoacoustic imager including a detection portion detecting an acoustic wave resulting from light applied to a specimen and an ultrasonic wave applied to and reflected by the specimen is known in general, as disclosed in Japanese Patent Laying-Open No. 2012-196308, for example.

The aforementioned Japanese Patent Laying-Open No. 2012-196308 discloses a photoacoustic image generator including an ultrasonic probe detecting a photoacoustic signal resulting from a laser beam applied to a specimen and a reflected acoustic signal applied to and reflected by the specimen. This photoacoustic image generator is provided with a light source unit including a Q-switched pulsed laser beam source, the ultrasonic probe and a signal processing portion. The photoacoustic image generator is configured to apply a laser beam from the light source unit to the specimen, to apply an ultrasonic wave from the ultrasonic probe to the specimen and to detect an acoustic signal and a reflected acoustic signal generated by the specimen with the ultrasonic probe. The signal processing portion is configured to generate a photoacoustic image and an ultrasonic image on the basis of the acoustic signal and the reflected acoustic signal detected by the ultrasonic probe.

An ultrasonic probe detecting an acoustic wave (an ultrasonic wave) is also generally known, as disclosed in "Medical Ultrasonic Equipment Handbook", compiled by Electric Industries Association of Japan, Corona Publishing Co., Ltd., April 1985, for example.

In general, an ultrasonic probe is so configured that the maximum frequency detectable by the same is at least 1 MHz and not more than 20 MHz. The photoacoustic image generator according to the aforementioned Japanese Patent Laying-Open No. 2012-196308 applies pulsed light (having a pulse width of 5 ns, for example) to the specimen with the Q-switched pulsed laser beam source. In this case, the specimen conceivably generates an acoustic wave of at least 0 Hz and not more than about 200 MHz. In other words, the photoacoustic image generator according to the aforementioned Japanese Patent Laying-Open No. 2012-196308 conceivably has such an inconvenience that the same causes excessive generation of an acoustic wave having a frequency higher than the maximum frequency detectable by the ultrasonic probe and not contributing to imaging of the acoustic wave (the specimen). Therefore, the photoacoustic image generator according to the aforementioned Japanese Patent Laying-Open No. 2012-196308 conceivably has such a problem that the same cannot make the specimen efficiently generate an acoustic wave due to the generation of the acoustic wave not contributing to the imaging.

SUMMARY OF THE INVENTION

The present invention has been proposed in order to solve the aforementioned problem, and an object of the present invention is to provide a photoacoustic imager capable of making a specimen efficiently generate an acoustic wave.

In order to attain the aforementioned object, a photoacoustic imager according to a first aspect of the present invention includes a light source portion applying pulsed light to a specimen and a detection portion detecting an acoustic wave generated by a detection object in the specimen absorbing the pulsed light applied from the light source portion to the specimen, and is configured to set the pulsed light to a pulse width tw expressed in the following expression (1), assuming that fmax represents the maximum frequency of the acoustic wave detected by the detection portion:

$$0.5/\mathrm{fmax} \leq \mathrm{tw} \leq 1/\mathrm{fmax} \ldots (1)$$

When the photoacoustic imager is so configured that the pulse width of the pulsed light applied from the light source portion to the specimen is at least 50 ns (0.5/fmax) and not more than 100 ns (1/fmax), for example, the maximum frequency of the acoustic wave generated by the specimen is about 10 MHz (fmax). In other words, the specimen generates an acoustic wave having a frequency of not more than 10 MHz (fmax).

The inventors have noted the aforementioned phenomenon, to reach the present invention. In other words, the light source portion is configured to set the pulsed light to the pulse width tw expressed in the above expression (1) in the photoacoustic imager according to the first aspect of the present invention as described above, whereby the maximum frequency of the acoustic wave generated by the specimen can be matched with the maximum frequency detectable by the detection portion. Consequently, the photoacoustic imager can prevent generation of an acoustic wave having a frequency higher than the maximum frequency detectable by the detection portion and not contributing to imaging, whereby the same can make the specimen efficiently generate an acoustic wave due to the prevention of generation of an acoustic wave not contributing to imaging.

The aforementioned photoacoustic imager according to the first aspect preferably further includes a light source driving portion driving the light source portion by supplying current to the light source portion, the light source portion preferably includes a light-emitting element emitting the pulsed light with the current supplied by the light source driving portion, and the photoacoustic imager is preferably so configured that the light source driving portion adjusts the time width of a pulse signal of the current flowing in the light-emitting element thereby setting the pulsed light to the pulse width tw expressed in the expression (1). According to this structure, the photoacoustic imager can set the pulsed light to the pulse width tw expressed in the above expression (1) with no requirement for a shutter (constituted of a plurality of EO (electrooptic) modulators and a polarizer or the like, for example) temporarily blocking the light.

The aforementioned photoacoustic imager according to the first aspect is preferably configured to set the pulsed light to the pulse width tw of at least 100 ns and not more than 500 ns. When a light-emitting element (a light-emitting diode or the like) is provided on the light source portion, for example, high current must be fed thereto and pulsed light must be set to a relatively large pulse width since the light-emitting element generally has a smaller quantity of light as compared with a solid laser. When the pulse width is excessively increased in the case of feeding high current to the light-emitting element, however, the light-emitting element may be deteriorated. When the photoacoustic imager is configured to set the pulsed light to the pulse width tw of at least 100 ns and not more than 500 ns as in the present invention, therefore, an acoustic wave necessary for performing imaging can be prevented from insufficiency and the light source portion (the light-emitting element) can also be prevented from deterioration, also in a case of employing a light source having a small quantity of light.

The aforementioned photoacoustic imager according to the first aspect preferably sets the frequency of the pulsed light to at least 100 Hz and not more than 100 kHz. If the frequency of the pulsed light is less than 100 Hz when the detection portion acquires the acoustic wave from the detection object and the photoacoustic imager images the acquired acoustic wave, the generated image may give an uncomfortable (unnatural) feeling to a human observer as a dynamic image. When the frequency of the pulsed light is rendered higher than 100 kHz, on the other hand, the quantity of the light emitted by the light source portion may exceed the MPE (maximum permissible exposure) on the skin of the specimen. In consideration of these points, the photoacoustic imager according to the present invention sets the frequency of the pulsed light to at least 100 Hz and not more than 100 kHz, whereby the same can display an image of the acoustic wave generated by the detection object as a dynamic image without giving an uncomfortable feeling to the human observer, and can also prevent the quantity of the light emitted by the light source portion from exceeding the MPE on the skin of the specimen.

In this case, the photoacoustic imager preferably sets the frequency of the pulsed light to at least 500 Hz and not more than 20 kHz. When the frequency of the pulsed light is rendered higher than 20 kHz, heat generation in the light source portion is conceivably relatively increased. With respect to this point, the photoacoustic imager according to the present invention can suppress heat generation in the light source portion by setting the frequency of the pulsed light to not more than 20 kHz. Further, the photoacoustic imager can display the image of the acoustic wave generated by the detection object as a dynamic image more without giving an uncomfortable feeling to the human observer by setting the frequency of the pulsed light to at least 500 Hz. Thus, the photoacoustic imager can display the image of the acoustic wave generated by the detection object as a dynamic image more without giving an uncomfortable feeling to the human observer while suppressing heat generation in the light source portion.

The aforementioned photoacoustic imager according to the first aspect preferably further includes a light source driving portion driving the light source portion by supplying current to the light source portion, the light source portion preferably includes a light-emitting element emitting the pulsed light with the current supplied by the light source driving portion, and the light source driving portion is preferably configured to supply the current to the light source portion so that the maximum value of the current flowing in the light-emitting element is at least the rated current of the light-emitting element and the density of the current flowing in the light-emitting element is not more than 120 A/mm$^2$. In general, the light-emitting element has a smaller quantity of light as compared with a solid laser, and hence an acoustic wave necessary for performing imaging may be insufficient also when the rated current is supplied to the light-emitting element for applying the pulsed light to the specimen. If the density of the current flowing in the light-emitting element is excessively increased, on the other hand, the light-emitting element may be deteriorated. When the light source driving portion is configured to supply the current to the light source portion so that the maximum value of the current flowing in the light-emitting element is at least the rated current of the light-emitting element and the density of the current flowing in the light-emitting element is not more than 120 A/mm² as in the present invention, therefore, the photoacoustic imager can more reliably prevent the light-emitting element from deterioration while suppressing insufficiency in the acoustic wave necessary for performing imaging. The aforementioned rated current denotes a predetermined reference value of current fed to the light-emitting element as direct current.

The aforementioned photoacoustic imager according to the first aspect preferably further includes a light source driving portion driving the light source portion by supplying current to the light source portion, a control portion controlling driving of the light source driving portion and a probe including the detection portion, and the control portion is preferably configured to control the driving of the light source driving portion so that the pulsed light emitted by the light source portion has the pulse width tw expressed in the expression (1) on the basis of information of the maximum frequency of the acoustic wave detected by the probe. According to this structure, the photoacoustic imager can set the pulsed light to a pulse width responsive to the maximum frequency detectable by each probe also when the probe is replaced with another probe capable of detecting a different maximum frequency or the photoacoustic imager is provided with a plurality of probes capable of detecting different maximum frequencies.

In this case, the photoacoustic imager preferably further includes an imager body portion having the control portion arranged therein and are so provided with a plurality of the probes that the maximum frequencies of the acoustic wave are different from each other, and the control portion is preferably configured to acquire information of the maximum frequencies of the acoustic wave when the probes are connected to the imager body portion. According to this structure, the photoacoustic imager can easily set the pulsed light to the pulse widths tw responsive to the maximum frequencies fmax of the acoustic wave detectable by the respective probes by connecting the probes to the imager body portion.

In the aforementioned photoacoustic imager having the control portion acquiring the information of the maximum frequencies of the acoustic wave when the probes are connected to the imager body portion, the probes preferably include storage portions storing the information of the maximum frequencies of the acoustic wave. According to this structure, the control portion can easily acquire the information of the maximum frequencies of the acoustic wave from the storage portions of the probes when the probes are connected to the imager body portion.

In the aforementioned photoacoustic imager according to the first aspect, the light source portion preferably includes a light-emitting diode element. According to this structure, the light-emitting diode element is lower in directivity as compared with a light-emitting element emitting a laser beam, and hence a light emission range remains relatively unchanged also when misregistration takes place. Thus, the photoacoustic imager requires neither precise alignment (registration) of optical members nor an optical platen or a strong housing for preventing characteristic fluctuation resulting from vibration of an optical system. Consequently, the photoacoustic imager can be prevented from size increase and complication in structure due to the nonrequirement for precise alignment of optical members and an optical platen or a strong housing.

In the aforementioned photoacoustic imager according to the first aspect, the light source portion preferably includes a semiconductor laser element. According to this structure, the photoacoustic imager can apply a laser beam relatively higher in directivity as compared with a light-emitting diode element to the specimen, whereby most part of the light from the semiconductor laser element can be reliably applied to the specimen.

In the aforementioned photoacoustic imager according to the first aspect, the light source portion preferably includes an organic light-emitting diode element. According to this structure, the light source portion including the organic light-emitting diode element can be easily miniaturized by employing the organic light-emitting diode element easily reducible in thickness.

The aforementioned photoacoustic imager according to the first aspect is preferably so configured that the maximum frequency fmax of the acoustic wave detected by the detection portion is at least 1 MHz and not more than 20 MHz. In general, resolution is increased as the frequency of the acoustic wave is increased, while an attenuation factor at a time when the acoustic wave is propagated in the specimen is also increased. When the specimen (an organism, for example) generates an acoustic wave having a frequency higher than 20 MHz, for example, it is difficult for an acoustic wave, having a frequency higher than 20 MHz, generated from a portion deeper than a surface layer portion to reach the detection portion. In other words, when the maximum frequency fmax detectable by the detection portion is rendered higher than 20 MHz, the frequency of the detected acoustic wave is equivalent to that in a case of setting the maximum frequency fmax detectable by the detection portion to 20 MHz. In general, further, the distance (depth) allowing propagation of the acoustic wave in the specimen is increased while the resolution is reduced as the frequency of the acoustic wave is reduced. In a case of performing imaging with an acoustic wave having a frequency of 1 MHz, for example, the resolution is about 1.5 mm. In a case of employing an image of an acoustic wave for diagnosis, the resolution is preferably not more than 1.5 mm in general. In consideration of these points, the photoacoustic imager according to the present invention is so configured that the maximum frequency fmax of the acoustic wave detected by the detection portion is at least 1 MHz and not more than 20 MHz, whereby the same can increase the detectable depth of the acoustic wave while increasing the resolution.

In the aforementioned photoacoustic imager according to the first aspect, the light source portion preferably includes a plurality of light-emitting element groups each formed by serially connecting light-emitting elements with each other, and the photoacoustic imager preferably further includes switch portions, provided for the light-emitting element groups respectively, having first sides connected to the light-emitting elements and grounded second sides. According to this structure, the photoacoustic imager can properly switch states of applying and not applying light from the light-emitting elements to the specimen with the switch portions provided for the light-emitting element groups respectively also when characteristics of forward voltage values are dispersed among the light-emitting element groups.

The aforementioned photoacoustic imager according to the first aspect preferably further includes a control portion configured to average a detection signal detected by the detection portion. According to this structure, the photoacoustic imager can improve the signal-to-noise ratio of the detection signal.

The aforementioned photoacoustic imager according to the first aspect preferably further includes a light source driving portion driving the light source portion by supplying current in the form of a triangular wave to the light source portion. The specimen (the detection object) generates the acoustic wave in response to change in the intensity of the light absorbed by the same. In consideration of this point, the photoacoustic imager according to the present invention includes the light source driving portion driving the light source portion by supplying the current in the form of a triangular wave thereto, whereby the same can apply pulsed light having a triangular waveform to the specimen. Thus, the photoacoustic imager can regularly change the intensity of the light absorbed by the detection object while applying the pulsed light to the specimen, whereby the same can make the detection object efficiently generate the acoustic wave.

In this case, the light source driving portion is preferably configured to supply the current in the form of a triangular wave, having a peak value of at least 15 A and not more than 75 A, to the light source portion. If the light source driving portion supplies current having a peak value less than 15 A to the light source portion for applying light to the specimen, the intensity of the acoustic wave generated by the detection object may be insufficient. If the light source driving portion supplies current having a peak value larger than 75 A to the light source portion for applying light to the specimen, on the other hand, the light source portion may be deteriorated. In consideration of these points, the light source driving portion is configured to supply the current in the form of a triangular wave having the peak value of at least 15 A and not more than 75 A to the light source portion according to the present invention, whereby the photoacoustic imager can prevent the light source portion from deterioration while suppressing insufficiency in the intensity of the acoustic wave generated by the detection object.

In the aforementioned photoacoustic imager according to the first aspect, the light source portion is preferably configured to emit pulsed light having a wavelength in the infrared region. According to this structure, the light having the wavelength in the infrared region relatively easily penetrates a human body, whereby the photoacoustic imager can deliver the light from the light source portion to a deeper portion of the specimen when the specimen is prepared from a human body.

A photoacoustic imager according to a second aspect of the present invention includes a light source portion applying pulsed light to a specimen and a detection portion detecting an acoustic wave generated by a detection object in the specimen absorbing the pulsed light applied from the light source portion to the specimen, and is configured to set the pulse width of the pulsed light to be reduced as the maximum frequency of the acoustic wave is increased and to be increased as the maximum frequency of the acoustic wave is reduced.

As hereinabove described, the photoacoustic imager according to the second aspect of the present invention sets the pulse width of the pulsed light to be reduced as the maximum frequency of the acoustic wave is increased and to be increased as the maximum frequency of the acoustic wave is reduced, whereby the same can also make the specimen efficiently generate the acoustic wave.

The aforementioned photoacoustic imager according to the second aspect is preferably configured to set the pulsed light to a pulse width tw expressed in the following expression (2), assuming that fmax represents the maximum frequency of the acoustic wave detected by the detection portion:

$$0.5/fmax \leq tw \leq 1/fmax \ldots (2)$$

According to this structure, the maximum frequency of the acoustic wave generated by the specimen can be matched with the maximum frequency detectable by the detection portion. Consequently, the photoacoustic imager can prevent generation of an acoustic wave having a frequency higher than the maximum frequency detectable by the detection portion and not contributing to imaging, whereby the same can make the specimen efficiently generate an acoustic wave due to the prevention of generation of an acoustic wave not contributing to imaging.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a block diagram showing the overall structure of a photoacoustic imager according to a first embodiment (a second embodiment) of the present invention;

Fig. 2 is a circuit diagram showing part of the structure of the photoacoustic imager according to the first embodiment (a third embodiment) of the present invention;

Fig. 3 shows a frequency characteristic (1) of an acoustic wave detectable by a detection portion according to the first embodiment of the present invention;

Fig. 4 is a diagram for illustrating structural examples of the maximum frequency detectable by the detection portion according to the first embodiment of the present invention and a set range of pulse widths;

Fig. 5 shows a frequency characteristic (2) of the acoustic wave detectable by the detection portion according to the first embodiment of the present invention;

Fig. 6 is a diagram for illustrating results of an experiment conducted for comparing the photoacoustic imager according to the first embodiment of the present invention and a photoacoustic imager according to comparative example with each other;

Fig. 7 is a timing chart for illustrating operations of the photoacoustic imager according to the second embodiment of the present invention;

Fig. 8 is a diagram for illustrating the relation between the size of a light-emitting diode element according to the third embodiment of the present invention and current density allowing deterioration of the light-emitting diode element;

Fig. 9 is a block diagram showing the overall structure of a photoacoustic imager according to a fourth embodiment of the present invention; and

Fig. 10 is a block diagram showing the overall structure of a photoacoustic imager according to each of first and second modifications of the first embodiment of the present invention.

DESCRIPTION OF THE PREFERRED EMBODIMENTS

Embodiments of the present invention are now described with reference to the drawings.

(First Embodiment)

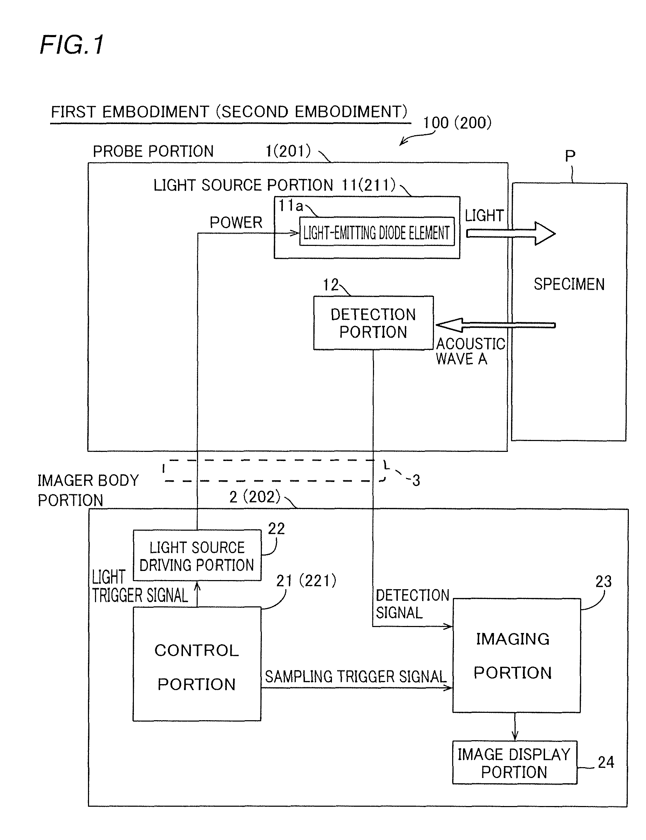

The structure of a photoacoustic imager 100 according to a first embodiment of the present invention is described with reference to Figs. 1 to 5.

The photoacoustic imager 100 according to the first embodiment of the present invention is provided with a probe portion 1 and an imager body portion 2, as shown in Fig. 1. The photoacoustic imager 100 is also provided with a cable 3 connecting the probe portion 1 and the imager body portion 2 with each other.

The probe portion 1 is so configured that the same is grasped by an operator and arranged on a surface of a specimen P (such as a surface of a human body). The probe portion 1 is configured to be capable of applying light to the specimen P, to detect an acoustic wave A, described later, generated by the specimen P and to transmit the acoustic wave A to the imager body portion 2 through the cable 3 as a detection signal.

The imager body portion 2 is configured to process and image the detection signal detected by the probe portion 1 and to display the imaged acoustic wave A.

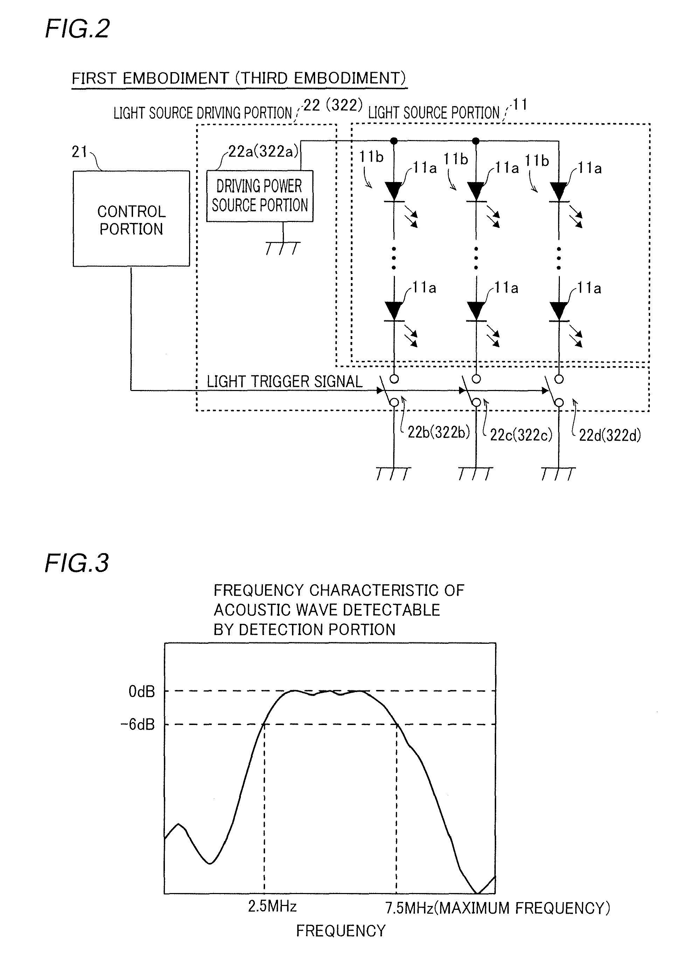

The probe portion 1 is provided with a light source portion 11. The light source portion 11 includes a plurality of light-emitting diode elements 11a. As shown in Fig. 2, the light-emitting diode elements 11a are arranged as a plurality of light-emitting element groups 11b each formed by serially connecting 36 light-emitting diode elements 11a with each other, for example, and three such light-emitting element groups 11b are parallelly connected to a light source driving portion 22 described later. In other words, the light source portion 11 is provided with 108 light-emitting diode elements 11a in total. The light-emitting diode elements 11a are examples of the "light-emitting element" in the present invention.

The light source portion 11 is configured to be capable of emitting pulsed light having a wavelength in the infrared region (a wavelength in the range of about 600 nm to about 1000 nm, such as a wavelength of about 850 nm or that of about 750 nm, for example) by being supplied with current from the light source driving portion 22. The light source portion 11 is also configured to apply light emitted from the plurality of light-emitting diode elements 11a to the specimen P.

A detection object (hemoglobin or the like, for example) in the specimen P absorbs the pulsed light applied from the probe portion 1 to the specimen P. The detection object (the specimen P) generates the acoustic wave A by expanding and contracting (returning to the original size from an expanding state) in response to the intensity of application (the quantity of absorption) of the pulsed light.

The probe 1 is also provided with a detection portion 12. The detection portion 12 is constituted of a piezoelectric element (lead zirconate titanate (PZT), for example) or the like. The detection portion 12 is configured to vibrate and generate voltage (the detection signal) when acquiring the aforementioned acoustic wave A. The detection portion 12 is also configured to transmit the detection signal to an imaging portion 23 described later.

The imager body portion 2 is provided with a control portion 21. The control portion 21 is configured to transmit a light trigger signal to the light source driving portion 22. The light source driving portion 22 is configured to drive the light source portion 21 to emit light in response to the light trigger signal. The control portion 21 is also configured to transmit a sampling trigger signal to the imaging portion 23.

The imager body portion 2 is also provided with the light source driving portion 22. The light source driving portion 22 includes a driving power source portion 22a and switch portions 22b to 22d, as shown in Fig. 2.

The driving power source portion 22a is configured to acquire power from an external power source (not shown) and to generate prescribed direct voltage with the acquired power. The driving power source portion 22 is connected to anode sides of the light-emitting element groups 11b of the light source portion 11, and configured to apply the prescribed direct voltage to anode sides of the light-emitting diode elements 11a.

First sides of the respective switch portions 22b to 22d are connected to cathode sides of the light-emitting diode elements 11a of the light source portion 11, while second sides thereof are grounded. The switch portions 22b to 22d include FETs (field-effect transistors), for example, and are configured to be capable of switching ON-and OFF-states on the basis of the pulsed light trigger signal received from the control portion 21 respectively. The photoacoustic imager 100 is so configured that voltage values on the cathode sides of the light-emitting diode elements 11a are lowered (the cathode sides are grounded) thereby causing potential difference between the anode sides and the cathode sides of the light-emitting diode elements 11a when the switch portions 22b to 22d are turned on so that current flows to the light-emitting diode elements 11a.

The imager body portion 2 is also provided with the imaging portion 23. The imaging portion 23 is configured to acquire the sampling trigger signal and the detection signal from the control portion 21 and the detection portion 12 respectively. The imaging portion 23 is also configured to generate a tomographic image based on the acoustic wave A on the basis of the sampling trigger signal and the detection signal acquired by the same and to output the generated image to an image display portion 24.

The imager body portion 2 is also provided with the image display portion 24. The image display portion 24 is constituted of a liquid crystal panel or the like, and configured to display the image received from the imaging portion 23.

According to the first embodiment, the light source portion 11 is so configured that the light source driving portion 22 adjusts the time width (pulse width) of a pulse signal of the current flowing in the light-emitting diode elements 11a thereby setting the pulsed light to a pulse width tw expressed in the following expression (3), assuming that fmax represents the maximum frequency of the acoustic wave A detectable by the detection portion 12:

$$0.5/\mathrm{fmax} \leq \mathrm{tw} \leq 1/\mathrm{fmax} \ldots (3)$$

As shown in Fig. 3, the maximum frequency fmax detectable by the detection portion 12 is decided by the frequency characteristic of the acoustic wave A detectable by the detection portion 12. Referring to Fig. 3, the maximum frequency fmax detectable by the detection portion 12 is 7.5 MHz, i.e., the higher one of the frequencies (7.5 MHz and 2.5 MHz) smaller by 6 dB (reaching -6 dB) with reference to a peak value (0 dB).

The control portion 21 is configured to perform control of transmitting the light trigger signal to the switch portions 22b to 22d of the light source driving portion 22 so that the pulse width tw of the pulsed light emitted from the light-emitting diode elements 11a is at least about 67 ns (= 0.5/7 MHz) and not more than about 1300 ns (= 1/7.5 MHz) (100 ns, for example).

For example, the control portion 21 is configured to perform control of turning on the switch portions 22b to 22d in consideration of fall times of the light-emitting diode elements 11a or the like. Thus, current having the pulse width tw flows in the light-emitting diode elements 11a, whereby the light-emitting diode elements 11a can emit pulsed light having the pulse width tw.

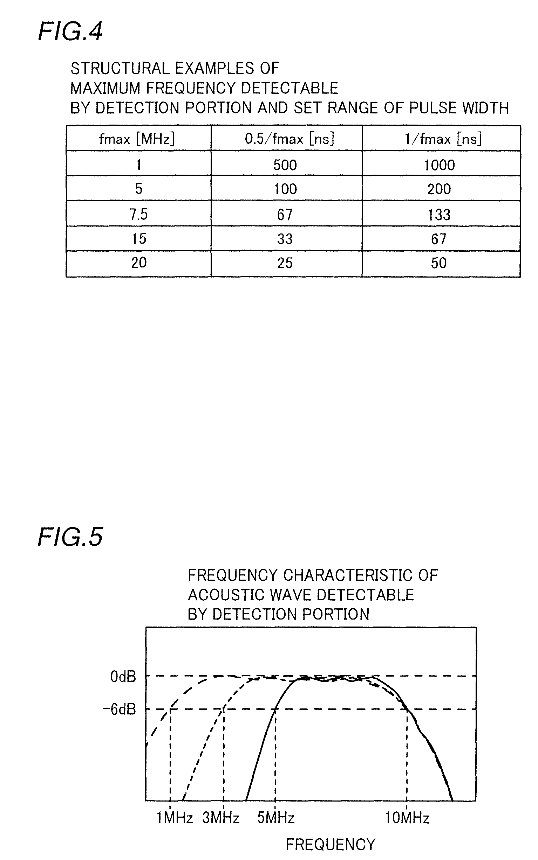

The photoacoustic imager 100 may be so configured that the maximum frequency fmax detectable by the detection portion 12 is other than 7.5 MHz, as shown in Fig. 4. For example, the photoacoustic imager 100 may be so configured that the maximum frequency fmax detectable by the detection portion 12 is 1 MHz, 5 MHz, 7.5 MHz, 15 MHz or 20 MHz.

The light source portion 11 is so configured that the pulse width tw of the pulsed light is reduced as the maximum frequency fmax detectable by the detection portion 12 is increased and the pulse width tw of the pulsed light is increased as the maximum frequency fmax detectable by the detection portion 12 is reduced, as shown in the above expression (3) and in Fig. 4.

The photoacoustic imager 100 is so configured that the maximum frequency fmax detectable by the detection portion 12 is not more than 20 MHz, as shown in Fig. 4. In general, resolution is increased as the frequency of the acoustic wave A is increased, while an attenuation factor at a time when the acoustic wave A is propagated in the specimen P is also increased. When the specimen P (an organism, for example) generates an acoustic wave A having a frequency higher than 20 MHz, for example, it is difficult for an acoustic wave A, having the frequency higher than 20 MHz, generated from a portion deeper than a surface layer portion to reach the detection portion 12. In other words, when the maximum frequency fmax detectable by the detection portion 12 is rendered higher than 20 MHz, the frequency of the detected acoustic wave A is equivalent to that of a case of setting the maximum frequency fmax detectable by the detection portion 12 to 20 MHz. Therefore, the photoacoustic imager 100 is preferably so configured that the maximum frequency fmax detectable by the detection portion 12 is not more than 20 MHz.

The photoacoustic imager 100 is so configured that the maximum frequency fmax detectable by the detection portion 12 is at least 1 MHz. In general, the distance (depth) allowing propagation of the acoustic wave A in the specimen P is increased while the resolution is reduced as the frequency of the acoustic wave A is reduced. In a case of performing imaging with the acoustic wave A of 1 MHz, for example, the resolution is about 1.5 mm. In a case of employing an image of the acoustic wave A for diagnosis, the resolution is preferably not more than 1.5 mm in general. Therefore, difficulty in diagnosis with the photoacoustic imager 100 can be prevented by configuring the detection portion 12 so that the maximum frequency fmax detectable by the same is at least 1 MHz.

The detection portion 12 may be so configured that the maximum frequency fmax detectable by the same is 10 MHz while the lower one (the minimum frequency) of frequencies reaching -6 dB with reference to the peak value (0 dB) is 5 MHz, 3 MHz or 1 MHz, as shown in Fig. 5.

In this case, the control portion 21 may be configured to perform such control that the pulse width tw of the pulsed light emitted from the light source portion 11 reaches any level in the range of at least 50 ns (= 0.5/fmax) and not more than 100 ns (= 1/fmax) in response to the magnitude of the minimum frequency detectable by the detection portion 12.

When the photoacoustic imager 100 is so configured that the maximum frequency fmax and the minimum frequency detectable by the detection portion 12 are 10 MHz and 1 MHz respectively, for example, the control portion 21 is so configured (set) that the pulse width tw of the pulsed light emitted from the light source portion 11 is 100 ns (= 1/fmax). When the photoacoustic imager 100 is so configured that the maximum frequency fmax and the minimum frequency detectable by the detection portion 12 are 10 MHz and 5 MHz respectively, on the other hand, the control portion 21 is so configured (set) that the pulse width tw of the pulsed light emitted from the light source portion 11 is 50 ns (= 0.5/fmax).

In this case, the specimen P generates an acoustic wave A having a frequency not more than 1/pulse width tw, as described later. When the minimum frequency is high as described above, therefore, the pulse width tw is set to a relatively small value so that the frequency characteristic of the acoustic wave A detectable by the detection portion 12 and the frequency distribution of the acoustic wave A generated by the specimen P can be matched with each other.

According to the first embodiment, the control portion 21 is configured to perform such control that a frequency (a sampling frequency (a repetition frequency)) for transmitting the light trigger signal to the light source driving portion 22 is at least 100 Hz and not more than 100 kHz. In other words, the frequency (the repetition frequency) of the pulsed light emitted from the light source portion 11 is set to at least 100 Hz and not more than 100 kHz. More preferably, the frequency (the repetition frequency) of the pulsed light emitted from the light source portion 11 is set to at least 500 Hz and not more than 20 kHz.

According to the first embodiment, the control portion 21 is also configured to average the detection signal detected by the detection portion 12 with the imaging portion 23. For example, the control portion 21 performs processing of averaging the detection signal 400 times by simple averaging or moving averaging. Thus, the control portion 21 can increase the signal-to-noise ratio of the detection signal 20 times.

When the control portion 21 averages the detection signal 400 times, for example, the frequency (the repetition frequency) of the pulsed light emitted from the light source portion 11 is so set to at least 100 Hz that an image (particularly an image with relatively small movement) generated by the imaging portion 23 hardly gives an uncomfortable (unnatural) feeling to a human observer as a dynamic image. When the frequency (the repetition frequency) of the pulsed light emitted from the light source portion 11 is set to at least 500 Hz, the image generated by the imaging portion 23 more hardly gives an uncomfortable (unnatural) feeling to the human observer as the dynamic image.

When the frequency (the repetition frequency) of the pulsed light emitted from the light source portion 11 is set to a larger value, the frequency of the pulsed light is so increased that the number of times of the averaging processing can be increased while maintaining (without reducing) a frame rate, which is the number of frames of the image processed per unit time.

When the specimen P is prepared from a human body, the MPE (maximum permissible exposure) on the skin of the specimen P is 29.29 J/$m^2$. When the light emitted from the light source portion 11 has a wavelength of 750 nm and a pulse width tw of 1000 ns, the period for applying the light is set to 0.5 s, the current flowing in the light-emitting diode elements 11a is set to 120 A/mm$^2$ and the frequency of the pulsed light is set to 100 kHz, peak power of the light is 7 kW, and energy per unit area is 28 J/m$^2$. In other words, the exposure of the light applied from the light source portion 11 to the skin of the specimen P is smaller than the MPE value.

The frequency (the repetition frequency) of the pulsed light emitted from the light source portion 11 is preferably set to not more than 20 kHz, so that heat generation in the light source portion 11 can be suppressed as compared with a case where the same is set to a value larger than 20 kHz.

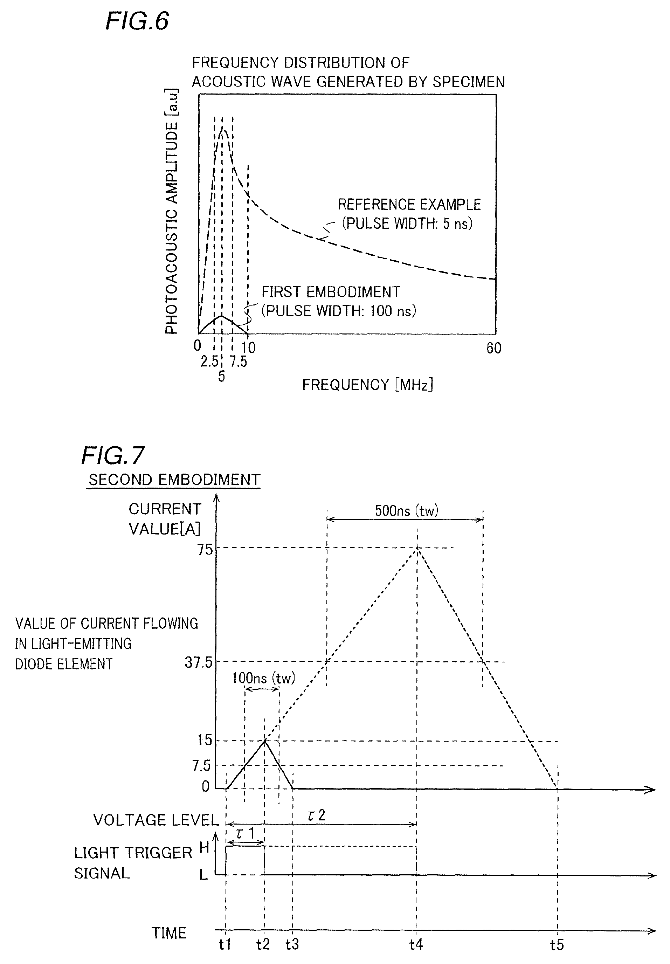

An experiment conducted for comparing frequency distributions of acoustic waves $\underline{A}$ generated by specimens P in the case (the first embodiment) of configuring the photoacoustic imager 100 to set the pulsed light to the pulse width tw expressed in the above expression (3) and a case (comparative example) of employing a photoacoustic imager prepared from a solid laser (a Q-switched pulsed laser beam source) and setting pulsed light to a pulse width of 5 ns and signal intensity levels detected by detection portions 12 is now described with reference to Fig. 6.

The detection portions 12 were so configured that maximum frequencies fmax were 7.5 MHz (see Fig. 3). Further, the photoacoustic imager 100 was so configured that the pulse width tw expressed in the above expression (3) was 100 ns in the aforementioned range of at least about 67 ns (= 0.5/7.5 MHz) and not more than about 133 ns (= 1/7.5 MHz).

In addition, peak power of the pulsed light emitted from the photoacoustic imager 100 according to the first embodiment was set to 1 kW, while that of pulsed light emitted from the photoacoustic imager according to comparative example was set to 1000 kW.

The frequency distributions of the acoustic waves $\underline{A}$ generated by the specimens P were measured by applying the pulsed light thereto. In the photoacoustic imager 100 according to the first embodiment, an acoustic wave $\underline{A}$ distributed in the range of at least 0 MHz and not more than 10 MHz (= 1/100 ns) was observed with a peak of 5 MHz. In the photoacoustic imager according to comparative example, on the other hand, an acoustic wave $\underline{A}$ distributed in the range of at least 0 MHz and not more than 200 MHz (= 1/5 ns) (frequencies higher than 60 MHz are not shown in Fig. 6) was observed with a peak of 5 MHz.

The signal intensity detected by the detection portion 12 of the photoacoustic imager according to comparative example was 20, assuming that the signal intensity detected by the detection portion 12 of the photoacoustic imager 100 according to the first embodiment was 1.

From the aforementioned results, it has been proved that the ratio of the signal intensity levels detected by the detection portions 12 of the photoacoustic imager 100 according to the first embodiment and the photoacoustic imager according to comparative example is 1:20, while the ratio of peak power levels of the pulsed light emitted by the photoacoustic imager 100 according to the first embodiment and the pulsed light emitted by the photoacoustic imager according to comparative example is 1:1000. In other words, it has been proved that the photoacoustic imager 100 according to the first embodiment can more efficiently make the specimen P generate the acoustic wave $\underline{A}$ by about 50 times (1000/20) as compared with the photoacoustic imager according to comparative example. This is conceivably because the efficiency of the photoacoustic imager according to comparative example is reduced due to generation of the acoustic wave $\underline{A}$, not contributing to the signal intensity detected by the detection portion 12, in the range larger than 7.5 MHz and not more than 200 MHz.

While the ratio of the signal intensity levels detected by the detection portions 12 of the photoacoustic imager 100 according to the first embodiment and the photoacoustic imager according to comparative example is 1:20, the control portion 21 of the photoacoustic imager 100 according to the first embodiment performs the processing of averaging the detection signal 400 times by simple averaging or moving averaging as described above, and hence the signal-to-noise ratio is increased 20 times. In other words, the signal-to-noise ratios of the photoacoustic imager 100 according to the first embodiment and the photoacoustic imager according to comparative example are substantially equal to each other.

From these results, it has been proved that the photoacoustic imager 100 according to the first embodiment can more efficiently make the specimen P generate the acoustic wave $\underline{A}$ by about 50 times (1000/20) as compared with the photoacoustic imager according to comparative example and that the signal-to-noise ratios of the photoacoustic imager 100 according to the first embodiment and the photoacoustic imager according to comparative example can be substantially equalized to each other.

According to the first embodiment, the following effects can be attained:

According to the first embodiment, as hereinabove described, the light source portion 11 is configured to set the pulsed light to the pulse width tw expressed in the above expression (3), whereby the maximum frequency of the acoustic wave $\underline{A}$ generated by the specimen P can be matched with the maximum frequency fmax detectable by the detection portion 12. Consequently, the photoacoustic imager 100 can suppress generation of an acoustic wave $\underline{A}$ having a frequency higher than the maximum frequency fmax detectable by the detection portion 12 and not contributing to imaging, whereby the same can make the specimen P efficiently generate the acoustic wave $\underline{A}$ due

to the suppression of generation of the acoustic wave A not contributing to imaging.

According to the first embodiment, as hereinabove described, the photoacoustic imager 100 is provided with the light source driving portion 22 driving the light source portion 11 by supplying current thereto. Further, the light source portion 11 is provided with the light-emitting diode elements 11a emitting pulsed light with the current received from the light source driving portion 22, and configured to set the pulsed light to the pulse width tw expressed in the above expression (3) by adjusting the time width of the pulse signal of the current fed by the light source driving portion 22 to the light-emitting diode elements 11a. Thus, the light source portion 11 can set the pulsed light to the pulse width tw expressed in the above expression (3) with no requirement for a shutter (constituted of a plurality of EO (electrooptic) modulators and a polarizer or the like, for example) temporarily blocking the light.

According to the first embodiment, as hereinabove described, the light source portion 11 is provided with the light-emitting diode elements 11a. The light-emitting diode elements 11a are lower in directivity as compared with light-emitting elements emitting laser beams, and hence a light emission range remains relatively unchanged also when misregistration takes place. Therefore, the photoacoustic imager 100 requires neither precise alignment (registration) of optical members nor an optical platen or a strong housing for preventing characteristic fluctuation resulting from vibration of an optical system, dissimilarly to a case of employing light-emitting elements emitting laser beams. Consequently, the photoacoustic imager 100 can be prevented from size increase and complication in structure due to the nonrequirement for precise alignment of optical members and an optical platen or a strong housing.

According to the first embodiment, as hereinabove described, the photoacoustic imager 100 sets the frequency of the pulsed light to at least 100 Hz and not more than 100 kHz. If the frequency of the pulsed light is less than 100 Hz when the detection portion 12 acquires the acoustic wave A from the detection object and the photoacoustic imager 100 images the acquired acoustic wave A, a generated image may give an uncomfortable (unnatural) feeling to the human observer as a dynamic image. If the frequency of the pulsed light is rendered higher than 100 kHz, on the other hand, the quantity of light emitted from the light source portion 11 may exceed the MPE (maximum permissible exposure) on the skin of the specimen P. In consideration of these points, the photoacoustic imager 100 according to the first embodiment sets the frequency of the pulsed light to at least 100 Hz and not more than 100 kHz, whereby the same can display an image of the acoustic wave A from the detection object as a dynamic image without giving an uncomfortable feeling to the human observer, and can also prevent the quantity of the light emitted by the light source portion 11 from exceeding the MPE on the skin of the specimen P.

According to the first embodiment, as hereinabove described, the photoacoustic imager 100 sets the frequency of the pulsed light to at least 500 Hz and not more than 20 kHz. When the frequency of the pulsed light is rendered higher than 20 kHz, heat generation in the light source portion 11 is conceivably relatively increased. With respect to this point, the photoacoustic imager 100 according to the first embodiment can suppress heat generation in the light source portion 11 by setting the frequency of the pulsed light to not more than 20 kHz. Further, the photoacoustic imager 100 can display the image of the acoustic wave A from the detection object as a dynamic image without giving an uncomfortable feeling to the human observer by setting the frequency of the pulsed light to at least 500 Hz. Thus, the photoacoustic imager 100 can display the image of the acoustic wave A from the detection object as a dynamic image more without giving an uncomfortable feeling to the human observer while suppressing heat generation in the light source portion 11.

According to the first embodiment, as hereinabove described, the detection portion 12 is so configured that the maximum frequency fmax of the acoustic wave A is at least 1 MHz and not more than 20 MHz. In general, the resolution is increased as the frequency of the acoustic wave A is increased, while the attenuation factor at the time when the acoustic wave A is propagated in the specimen P is also increased. When the specimen P (an organism, for example) generates an acoustic wave A having a frequency higher than 20 MHz, for example, it is difficult for the acoustic wave A, having the frequency higher than 20 MHz, generated from the portion deeper than the surface layer portion to reach the detection portion 12. In other words, when the maximum frequency fmax detectable by the detection portion 12 is rendered higher than 20 MHz, the frequency of the detected acoustic wave A is equivalent to that in the case of setting the maximum frequency fmax detectable by the detection portion 12 to 20 MHz. In general, further, the distance (depth) allowing propagation of the acoustic wave A in the specimen P is increased while the resolution is reduced as the frequency of the acoustic wave A is reduced. In a case of performing imaging with an acoustic wave A having a frequency of 1 MHz, for example, the resolution is about 1.5 mm. In the case of employing the image of the acoustic wave A for diagnosis, the resolution is preferably not more than 1.5 mm in general. In consideration of these points, the photoacoustic imager 100 according to the first embodiment is configured to set the maximum frequency fmax of the acoustic wave A detectable by the detection portion 12 to at least 1 MHz and not more than 20 MHz, whereby the same can increase the detectable depth of the acoustic wave A while increasing the resolution.

According to the first embodiment, as hereinabove described, the light source portion 11 is constituted of the plurality of light-emitting element groups 11b each formed by serially connecting the light-emitting diode elements 11a with each other, and the photoacoustic imager 100 further includes the switch portions 22b to 22d having the first sides connected to the light-emitting elements 11a and the grounded second sides. Thus, the photoacoustic imager 100 can properly switch states of applying and not applying light from the light-emitting elements 11a to the specimen P with the switch portions (22b, 22c or 22d) provided for the light-emitting element groups 11b respectively also when characteristics of forward voltage values are dispersed among the light-emitting element groups 11b.

According to the first embodiment, as hereinabove described, the control portion 21 is configured to average the detection signal detected by the detection portion 12. Thus, the photoacoustic imager 100 can improve the signal-to-noise ratio of the detection signal.

According to the first embodiment, as hereinabove described, the light source portion 11 is configured to emit the pulsed light having the wavelength in the infrared region. Thus, the light having the wavelength in the infrared region relatively easily penetrates a human body, whereby the photoacoustic imager 100 can deliver the light from the light source portion 11 to a deeper portion of the specimen P when the specimen P is prepared from a human body.

(Second Embodiment)

The structure of a photoacoustic imager 200 according to a second embodiment of the present invention is now described with reference to Figs. 1 and 7. According to the second embodiment, a light source portion 211 is configured to set pulsed light emitted therefrom to a pulse width tw of at least 100 ns and not more than 500 ns.

As shown in Fig. 1, the photoacoustic imager 200 according to the second embodiment is provided with a probe portion 201 and an imager body portion 202. The probe portion 201 includes the light source portion 211, while the imager body portion 202 includes a control portion 221. The light source portion 221 is provided therein with 108 light-emitting diode elements 11a, similarly to the light source portion 11 according to the first embodiment.

According to the second embodiment, the control portion 221 is configured to perform control of transmitting a light trigger signal to a light source driving portion 22 for a time of at least a period $\tau 1$ (times t1 to t2) and not more than a period $\tau 2$ (times t1 to t4) so that pulsed light emitted from the light source portion 211 (the light-emitting diode elements 11a) has the pulse width tw of at least 100 ns and not more than 500 ns. When the light trigger signal and the pulse width tw of the pulsed light are substantially identical to each other (in a case of employing light-emitting elements having an extremely high response speed, for example), the periods $\tau 1$ and $\tau 2$ may be set to about 100 ns and about 500 ns respectively.

The light-emitting diode elements 11a are so configured that current in the form of a triangular wave flows therein and the peak of the current value is 15 A when the light-emitting diode elements 11a are supplied with current from the light source driving portion 22 to emit pulsed light having a pulse width tw of 100 ns. In this case, the light source portion 211 is provided with 108 light-emitting diode elements 11a as described above, and hence peak power for making the light source portion 211 emit light is about 1 kW in total. The light source portion 211 preferably applies pulsed light having peak power of at least 1 kW to a specimen P so that an imaging portion 23 acquires a sufficient acoustic wave A for imaging the interior of the specimen P. Therefore, the photoacoustic imager 200 according to the second embodiment is so configured that the pulse width tw of the pulsed light is at least 100 ns, whereby the imaging portion 23 is configured to be capable of imaging the interior of the specimen P by acquiring a sufficient acoustic wave A.

The light-emitting diode elements 11a are so configured that current in the form of a triangular wave flows therein and the peak of the current value is 75 A when the light-emitting diode elements 11a are supplied with current from the light source driving portion 23 to emit pulsed light having a pulse width tw of 500 ns. The control portion 221 is configured to perform such control that a frequency (a sampling frequency (a repetition frequency)) for transmitting the light trigger signal to the light source driving portion 22 is 1 kHz. When current having a peak value larger than 75 A is fed to the light-emitting diode elements 11a, the light-emitting diode elements 11a may be deteriorated. Therefore, the photoacoustic imager 200 according to the second embodiment is so configured that the pulse width tw of the pulsed light is not more than 500 ns, whereby the same can prevent the light-emitting diode elements 11a from deterioration.

The remaining structures of the photoacoustic imager 200 according to the second embodiment are similar to those of the photoacoustic imager 100 according to the first embodiment.

Operations of the photoacoustic imager 200 according to the second embodiment are now described with reference to Fig. 7. In the photoacoustic imager 200, the control portion 221 performs control processing.

(When Pulse Width tw is 100 ns)

In the period $\tau 1$ (times t1 to t2), the control portion 221 sets the light trigger signal to a high voltage level. Then, the value of current flowing in the light-emitting diode elements 11a substantially linearly rises. At the time t2, the value of the current flowing in the light-emitting diode elements 11a reaches 15 A. At the time t2, further, the control portion 221 sets the light trigger signal to a low voltage level. Thereafter the value of the current flowing in the light-emitting diode elements 11a substantially linearly lowers. At the time t3, the value of the current flowing in the light-emitting diode elements 11a reaches substantially zero. The pulse width tw reaches 100 ns.

(When Pulse Width tw is 500 ns)

In the period τ2 (times t1 to t4), the control portion 221 set the light trigger signal to a high voltage level. Then, the value of current flowing in the light-emitting diode elements 11a substantially linearly rises. At the time t4, the value of the current flowing in the light-emitting diode elements 11a reaches 75 A. At the time t2, the control portion 221 sets the light trigger signal to a low voltage level. Thereafter the value of the current flowing in the light-emitting diode elements 11a substantially linearly lowers. At the time t5, the value of the current flowing in the light-emitting diode elements 11a reaches substantially zero. The pulse width tw reaches 500 ns.

According to the second embodiment, the following effects can be attained:

According to the second embodiment, as hereinabove described, the light source portion 211 is configured to set the pulsed light to the pulse width tw of at least 100 ns and not more than 500 ns. In general, the light-emitting diode elements 11a have small quantities of light as compared with solid lasers, and hence high current must be fed thereto and the pulse width of the pulsed light must be relatively increased. When the pulse width is excessively increased in the case of feeding high current, however, the light-emitting diode elements 11a may be deteriorated. When the photoacoustic imager 200 is configured to set the pulsed light to the pulse width tw of at least 100 ns and not more than 500 ns as in the second embodiment, therefore, an acoustic wave A necessary for performing imaging can be prevented from insufficiency and the light-emitting diode elements 11a can also be prevented from deterioration also in the case of employing the light-emitting diode elements 11a.

According to the second embodiment, as hereinabove described, the light source driving portion 22 is configured to drive the light source portion 211 by supplying the current in the form of a triangular wave thereto. The specimen P (a detection object) generates the acoustic wave A in response to change in the intensity of the light absorbed by the same. In consideration of this point, the light source driving portion 22 of the photoacoustic imager 200 according to the second embodiment drives the light source portion 211 by supplying the current in the form of a triangular wave thereto, whereby the light source portion 211 can apply pulsed light having a triangular waveform to the specimen P. Thus, the photoacoustic imager 200 can regularly change the intensity of the light absorbed by the detection object while applying the pulsed light to the specimen P, whereby the same can make the detection object efficiently generate the acoustic wave A.

According to the second embodiment, as hereinabove described, the light source driving portion 22 is configured to supply the current, in the form of a triangular wave, having the peak value of at least 15 A and not more than 75 A to the light source portion 211. If the light source driving portion 22 supplies current having a peak value less than 15 A to the light source portion f211 for applying light to the specimen P, the intensity of the acoustic wave A generated by the detection object may be insufficient. If the light source driving portion 22 supplies current having a peak value larger than 75 A to the light source portion 211 for applying light to the specimen P, on the other hand, the light source portion 211 may be deteriorated. In consideration of these points, the light source driving portion 22 is configured to supply the current, in the form of a triangular wave, having the peak value of at least 15 A and not more than 75 A to the light source portion 211 in the photoacoustic imager 200 according to the second embodiment, whereby the photoacoustic imager 200 can prevent the light source portion 211 from deterioration while suppressing insufficiency in the intensity of the acoustic wave A generated by the detection object.

The remaining effects of the photoacoustic imager 200 according to the second embodiment are similar to those of the photoacoustic imager 100 according to the first embodiment.

(Third Embodiment)

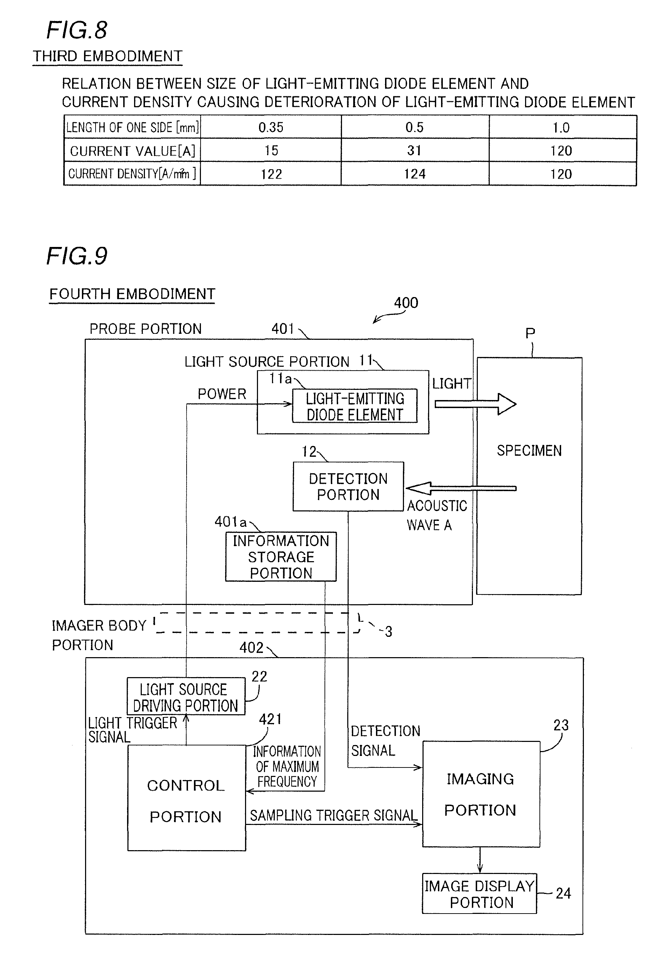

The structure of a photoacoustic imager 300 according to a third embodiment of the present invention is now described with reference to Figs. 2 and 8. According to the third embodiment, a light source driving portion 322 is configured to supply current to a light source portion 11 so that the maximum value of current flowing in light-emitting diode elements 11a is at least the rated current of the light-emitting diode elements 11a and the density thereof is not more than 120 A/mm$^2$.

As shown in Fig. 2, the photoacoustic imager 300 according to the third embodiment is provided with a control portion 321 and the light source driving portion 322.

According to the third embodiment, the light source driving portion 322 is configured to supply current to the light source portion 11 so that the maximum value of the current flowing in the light-emitting diode elements 11a is at least the rated current of the light-emitting diode elements 11a and the density thereof is not more than 120 A/mm$^2$. More specifically, the light source driving portion 322 includes a driving power source portion 322a and switch portions 322b to 322d, and is configured to drive the light source portion 11 to emit pulsed light having a pulse width tw based on the above expression (3) with the switch portions 322b to 322d on the basis of a light trigger signal received from the control portion 321. The driving power source portion 322a is configured to supply such voltage (and current) that the current flowing in the light-emitting diode elements 11a exceeds the rated current and the density thereof is not more than 120 A/mm$^2$.

An experiment conducted for investigating the relation between sizes of the light-emitting diode elements 11a in the photoacoustic imager 300 according to the third embodiment and current density causing deterioration of the light-emitting diode elements 11a is now described with reference to Fig. 8.

First, three square light-emitting diode elements 11a having side lengths of 0.35 mm, 0.5 mm and 1 mm respectively were employed. Minimum current values causing deterioration of the light-emitting diode elements 11a were investigated by feeding current (single pulse) having a pulse width of 1000 ns to the light-emitting diode elements 11a.

The light-emitting diode element 11a having the side length of 0.35 mm was deteriorated with current having a value higher than 15 A, the light-emitting diode element 11a having the side length of 0.5 mm was deteriorated with current having a value higher than 31 A, and the light-emitting diode element 11a having the side length of 1 mm was deteriorated with current having a value higher than 120 A.

Therefore, the light-emitting diode element 11a having the side length of 0.35 mm was deteriorated with current density higher than 122 A/mm$^2$, the light-emitting diode element 11a having the side length of 0.5 mm was deteriorated with current density higher than 124 A/mm$^2$, and the light-emitting diode element 11a having the side length of 1 mm was deteriorated with current density higher than 120 A/mm$^2$.

From the aforementioned results, it has been proved that the light-emitting diode elements 11a are deteriorated with current density higher than 120 A/mm$^2$, regardless of the sizes thereof.

The remaining structures of the photoacoustic imager 300 according to the third embodiment are similar to those of the photoacoustic imager 100 according to the first embodiment.

According to the third embodiment, the following effect can be attained:

According to the third embodiment, as hereinabove described, the light source driving portion 322 is configured to supply current to the light source portion 11 so that the maximum value of the current flowing in the light-emitting diode elements 11a is at least the rated current of the light-emitting diode elements 11a and the density thereof is not more than 120 A/mm$^2$. In general, the light-emitting diode elements 11a have smaller quantities of light as compared with solid lasers, and hence an acoustic wave $\underline{A}$ necessary for performing imaging may be insufficient also when rated current is supplied to the light-emitting diode elements 11a for applying the pulsed light to the specimen P. When the density of the current flowing in the light-emitting diode elements 11a is excessively increased, on the other hand, the light-emitting diode elements 11a may be deteriorated. When the light source driving portion 322 is configured to supply the current to the light source portion 11 so that the maximum value of the current flowing in the light-emitting diode elements 11a is at least the rated current of the light-emitting diode elements 11a and the density of the current flowing in the light-emitting diode elements 11a is not more than 120 A/mm$^2$ as in the third embodiment, therefore, the photoacoustic imager 300 can more reliably prevent the light-emitting diode elements 11a from deterioration while suppressing insufficiency in the acoustic wave $\underline{A}$ necessary for performing imaging.

The remaining effects of the photoacoustic imager 300 according to the third embodiment are similar to those of the photoacoustic imager 100 according to the first embodiment.

(Fourth Embodiment)

The structure of a photoacoustic imager 400 according to a fourth embodiment of the present invention is now described with reference to Fig. 9. According to the fourth embodiment, a control portion 421 is configured to acquire information of maximum frequencies fmax detectable by a plurality of probe portions 401 and to control driving of a light source driving portion 22 so that pulsed light emitted by a light source portion 11 has the pulse width tw expressed in the above expression (3) on the basis of the acquired information of the maximum frequencies fmax detectable by the probe portions 401.

As shown in Fig. 9, the photoacoustic imager 400 according to the fourth embodiment is provided with the probe portions 401 and an imager body portion 402. The probe portions 401 include information storage portions 401a, while the imager body portion 402 includes the control portion 421. The information storage portions 401a are examples of the "storage portion" in the present invention.

The probe portions 401 are configured to be attachable/detachable to/from the imager body portion 402 (a cable 3). The plurality of probe portions 401 are so provided that maximum frequencies of acoustic waves $\underline{A}$ are different from each other. The information storage portions 401a store information of the maximum frequencies fmax detectable by the probe portions 401. The control portion 421 is configured to acquire the information of the maximum frequencies fmax detectable by the probe portions 401 when the probe portions 401 are connected to the imager body portion 402, for example. The control portion 421 is also configured to control driving of the light source driving portion 22 so that pulsed light emitted by the light source portion 11 has the pulse width tw expressed in the above expression (3) on the basis of the acquired information of the maximum frequencies fmax detectable by the probe portions 401.

The remaining structures of the photoacoustic imager 400 according to the fourth embodiment are similar to those of the photoacoustic imager 100 according to the first embodiment.

According to the fourth embodiment, the following effects can be attained:

According to the fourth embodiment, as hereinabove described, the control portion 421 is configured to control driving of the light source driving portion 22 so that the pulsed light emitted by the light source portion 11 has the pulse width tw expressed in the above expression (3) on the basis of the acquired information of the maximum frequencies fmax of the acoustic waves $\underline{A}$ detectable by the probe portions 401. Thus, the control portion 421 can set the pulsed light to pulse widths tw responsive to the maximum frequencies fmax of the acoustic waves $\underline{A}$ detectable by the respective probe portions 401 also when the probe portions 401 are replaced with those capable of detecting different maximum frequencies fmax or the photoacoustic imager 400 is provided with the plurality of probe portions 401 capable of detecting maximum frequencies fmax different from each other.

According to the fourth embodiment, as hereinabove described, the imager body portion 402 is so configured that the control portion 421 is arranged therein, and the photoacoustic imager 400 is so provided with the plurality of probe portions 401 that the maximum frequencies fmax of the acoustic waves $\underline{A}$ detectable by the same are different from each other. Further, the control portion 421 is configured to acquire the information of the maximum frequencies fmax of the acoustic waves $\underline{A}$ when the probe portions 401 are connected to the imager body portion 402. Thus, the photoacoustic imager 400 can easily set the pulsed light to the pulse widths tw responsive to the maximum frequencies fmax of the acoustic waves $\underline{A}$ detectable by the respective probe portions 401 by connecting the probe portions 401 to the imager body portion 402.

According to the fourth embodiment, as hereinabove described, the probe portions 401 include the information storage portions 401a storing the information of the maximum frequencies fmax of the acoustic waves $\underline{A}$. Thus, the control portion 421 can easily acquire the information of the maximum frequencies fmax of the acoustic waves $\underline{A}$ from the information storage portions 401a of the probe portions 401 when the probe portions 401 are connected to the imager body portion 402.

The remaining effects of the photoacoustic imager 400 according to the fourth embodiment are similar to those of the photoacoustic imager 100 according to the first embodiment.

Although the present invention has been described and illustrated in detail, it is clearly understood that the same is by way of illustration and example only and is not to be taken by way of limitation, the spirit and scope of the present invention being limited only by the terms of the appended claims.

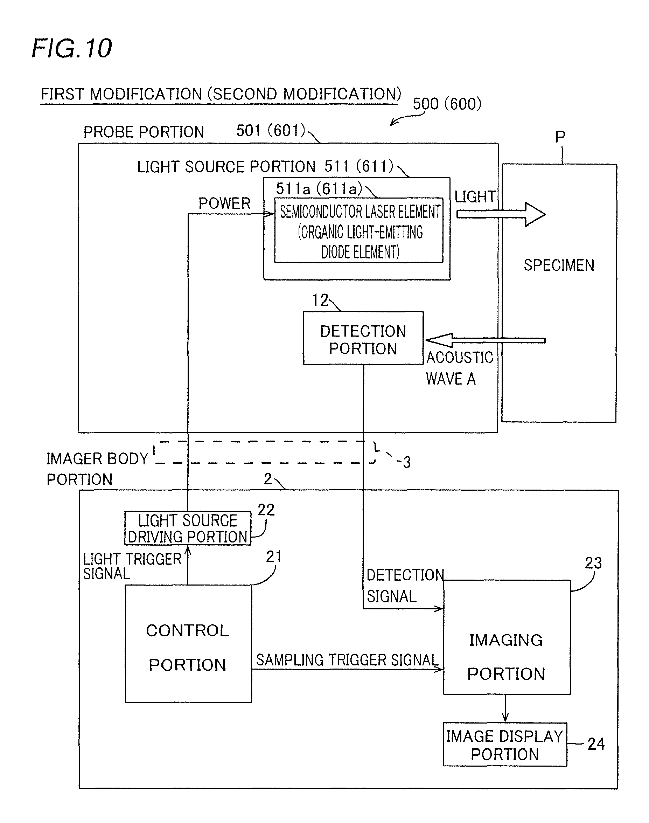

For example, while the light-emitting diode elements are employed as light-emitting elements in each of the aforementioned first to fourth embodiments, the present invention is not restricted to this. According to the present invention, light-emitting elements other than the light-emitting diode elements may alternatively be employed. For example, semiconductor laser elements 511a or organic light-emitting diode elements 611a may be employed as in a first or second modification shown in Fig. 10.

A photoacoustic imager 500 according to the first modification is provided with a probe portion 501, as shown in Fig. 10. The probe portion 501 includes a light source portion 511, which in turn includes the semiconductor laser elements 511a. The semiconductor laser elements 511a are configured to be capable of applying light to a specimen P. In this case, the semiconductor laser elements 511a can apply laser beams relatively higher in directivity as compared with light-emitting diode elements to the specimen P, whereby the photoacoustic imager 500 can reliably apply most part of the light from the semiconductor laser elements 511a to the specimen P.

On the other hand, a photoacoustic imager 600 according to the second modification is provided with a probe portion 601, as shown in Fig. 10. The probe portion 601 includes a light source portion 611, which in turn includes the organic light-emitting diode elements 611a. The organic light-emitting diode elements 611a are configured to be capable of applying light to a specimen P. In this case, the organic light-emitting diode elements 611a are easily reducible in thickness, whereby the light source portion 611 can be easily miniaturized.

While the detection portion capable of detecting the maximum frequency fmax of at least 1 MHz and not more than 20 MHz is employed in each of the aforementioned first to fourth embodiments, the present invention is not restricted to this. According to the present invention, a detection portion capable of detecting a maximum frequency fmax in a range other than that of at least 1 MHz and not more than 20 MHz may alternatively be employed. In other words, a detection portion capable of detecting a maximum frequency fmax less than 1 MHz or larger than 20 MHz may be employed.

While the photoacoustic imager is so configured that the light source driving portion adjusts the time width of the pulse signal of the current flowing in the light-emitting diode elements thereby setting the pulsed light to the pulse width tw expressed in the above expression (3) in each of the aforementioned first to fourth embodiments, the present invention is not restricted to this. According to the present invention, the photoacoustic imager may alternatively be configured to set the pulsed light to the pulse width tw expressed in the above expression (3) not with the operation of the light source driving portion adjusting the time width of the pulse signal of the current flowing in the light-emitting diode elements. For example, the photoacoustic imager may be provided with a shutter capable of blocking light, for setting the pulsed light to the pulse width tw expressed in the above expression (3).

While the light source driving portion is provided with three switch portions in each of the aforementioned first to fourth embodiments, the present invention is not restricted to this. According to the present invention, the light source driving portion may alternatively be provided with switch portions in a number other than three. When characteristics of forward voltage values of the light-emitting diode elements are substantially uniform

(not remarkably dispersed), the light source driving portion may be provided with one switch portion connected with the cathode sides of the three light-emitting element groups.

While the light source portion is provided with 108 light-emitting diode elements in each of the aforementioned first to fourth embodiments, the present invention is not restricted to this. According to the present invention, the light source portion may alternatively be provided with light-emitting diode elements in a number other than 108. In other words, the light source portion may be provided with light-emitting diode elements in a number less than or larger than 108.

**Claims**

1. A photoacoustic imager (100, 200, 300, 400, 500, 600) comprising:

   a light source portion (11, 211, 511, 611) applying pulsed light to a specimen; and
   a detection portion (12) detecting an acoustic wave generated by a detection object in the specimen absorbing the pulsed light applied from the light source portion to the specimen, and
   configured to set the pulsed light to a pulse width tw expressed in the following expression (1), assuming that fmax represents the maximum frequency of the acoustic wave detected by the detection portion:

$$0.5/fmax \leq tw \leq 1/fmax \ldots (1)$$

2. The photoacoustic imager according to claim 1, further comprising a light source driving portion (22, 322) driving the light source portion by supplying current to the light source portion, wherein
   the light source portion includes a light-emitting element (11a, 511a, 611a) emitting the pulsed light with the current supplied by the light source driving portion, and
   the photoacoustic imager is so configured that the light source driving portion adjusts the time width of a pulse signal of the current flowing in the light-emitting element thereby setting the pulsed light to the pulse width tw expressed in the expression (1).

3. The photoacoustic imager according to claim 1 or 2, configured to set the pulsed light to the pulse width tw of at least 100 ns and not more than 500 ns.

4. The photoacoustic imager according to any of claims 1 to 3, setting the frequency of the pulsed light to at least 100 Hz and not more than 100 kHz.

5. The photoacoustic imager according to claim 4, setting the frequency of the pulsed light to at least 500 Hz and not more than 20 kHz.

6. The photoacoustic imager according to any of claims 1 to 5, further comprising a light source driving portion (22, 322) driving the light source portion by supplying current to the light source portion, wherein
   the light source portion includes a light-emitting element (11a, 511a, 611a) emitting the pulsed light with the current supplied by the light source driving portion, and
   the light source driving portion is configured to supply the current to the light source portion so that the maximum value of the current flowing in the light-emitting element is at least the rated current of the light-emitting element and the density of the current flowing in the light-emitting element is not more than 120 A/mm$^2$.

7. The photoacoustic imager according to any of claims 1 to 6, further comprising:

a light source driving portion (22, 322) driving the light source portion by supplying current to the light source portion,

a control portion (21, 221, 321, 421) controlling driving of the light source driving portion, and

a probe (1, 201, 401, 501, 601) including the detection portion, wherein

the control portion is configured to control the driving of the light source driving portion so that the pulsed light emitted by the light source portion has the pulse width tw expressed in the expression (1) on the basis of information of the maximum frequency of the acoustic wave detected by the probe.

8. The photoacoustic imager according to claim 7, further comprising an imager body portion (2, 202, 402) having the control portion arranged therein, and

so provided with a plurality of the probes that the maximum frequencies of the acoustic wave are different from each other, wherein

the control portion is configured to acquire information of the maximum frequencies of the acoustic wave when the probes are connected to the imager body portion.

9. The photoacoustic imager according to claim 8, wherein

the probes include storage portions (401a) storing the information of the maximum frequencies of the acoustic wave.

10. The photoacoustic imager according to any of claims 1 to 9, wherein

the light source portion includes a light-emitting diode element (11a).

11. The photoacoustic imager according to any of claims 1 to 10, wherein

the light source portion includes a semiconductor laser element (511a).

12. The photoacoustic imager according to any of claims 1 to 11, wherein

the light source portion includes an organic light-emitting diode element (611a).

13. The photoacoustic imager according to any of claims 1 to 12, so configured that the maximum frequency fmax of the acoustic wave detected by the detection portion is at least 1 MHz and not more than 20 MHz.

14. The photoacoustic imager according to any of claims 1 to 13, wherein

the light source portion includes a plurality of light-emitting element groups (11b) each formed by serially connecting light-emitting elements (11a, 511a, 611a) with each other, and

the photoacoustic imager further comprises switch portions (22b - 22d), provided for the light-emitting element groups respectively, having first sides connected to the light-emitting elements and grounded second sides.

15. The photoacoustic imager according to any of claims 1 to 14, further comprising a control portion (21, 221, 321, 421) configured to average a detection signal detected by the detection portion.

## FIG.1

FIRST EMBODIMENT (SECOND EMBODIMENT)    100 (200)

PROBE PORTION    1(201)

LIGHT SOURCE PORTION 11(211)

11a

POWER → LIGHT-EMITTING DIODE ELEMENT    LIGHT →    P

SPECIMEN

12

DETECTION PORTION ← ACOUSTIC WAVE A

IMAGER BODY PORTION    3

2 (202)

22

LIGHT SOURCE DRIVING PORTION

LIGHT TRIGGER SIGNAL    21 (221)    DETECTION SIGNAL    23

CONTROL PORTION    SAMPLING TRIGGER SIGNAL    IMAGING PORTION

IMAGE DISPLAY PORTION    24

## FIG.2

FIRST EMBODIMENT (THIRD EMBODIMENT)

LIGHT SOURCE DRIVING PORTION ⁄ 22 (322)  LIGHT SOURCE PORTION ⁄ 11

21

CONTROL PORTION

22a(322a)

DRIVING POWER SOURCE PORTION

11b   11a   11b   11a   11b   11a

11a   11a   11a

LIGHT TRIGGER SIGNAL

22b(322b)   22c(322c)   22d(322d)

## FIG.3

FREQUENCY CHARACTERISTIC OF ACOUSTIC WAVE DETECTABLE BY DETECTION PORTION

0dB

-6dB

2.5MHz        7.5MHz(MAXIMUM FREQUENCY)

FREQUENCY

## FIG.4

STRUCTURAL EXAMPLES OF
MAXIMUM FREQUENCY DETECTABLE
BY DETECTION PORTION AND SET RANGE OF PULSE WIDTH

| fmax [MHz] | 0.5/fmax [ns] | 1/fmax [ns] |
|------------|---------------|-------------|
| 1 | 500 | 1000 |
| 5 | 100 | 200 |
| 7.5 | 67 | 133 |
| 15 | 33 | 67 |
| 20 | 25 | 50 |

## FIG.5

FREQUENCY CHARACTERISTIC OF
ACOUSTIC WAVE DETECTABLE
BY DETECTION PORTION

## FIG.6

FREQUENCY DISTRIBUTION OF
ACOUSTIC WAVE GENERATED BY SPECIMEN

REFERENCE EXAMPLE
(PULSE WIDTH: 5 ns)

FIRST EMBODIMENT
(PULSE WIDTH: 100 ns)

PHOTOACOUSTIC AMPLITUDE [a.u]

FREQUENCY [MHz]

## FIG.7

SECOND EMBODIMENT

## FIG.8

THIRD EMBODIMENT

RELATION BETWEEN SIZE OF LIGHT-EMITTING DIODE ELEMENT AND
CURRENT DENSITY CAUSING DETERIORATION OF LIGHT-EMITTING DIODE ELEMENT

| LENGTH OF ONE SIDE [mm] | 0.35 | 0.5 | 1.0 |
|---|---|---|---|
| CURRENT VALUE[A] | 15 | 31 | 120 |
| CURRENT DENSITY[A/mm²] | 122 | 124 | 120 |

## FIG.9

FOURTH EMBODIMENT

## FIG.10

FIRST MODIFICATION (SECOND MODIFICATION)   500 (600)

PROBE PORTION   501 (601)

LIGHT SOURCE PORTION  511 (611)

511a (611a)

POWER → | SEMICONDUCTOR LASER ELEMENT (ORGANIC LIGHT-EMITTING DIODE ELEMENT) | → LIGHT → SPECIMEN

P

12

| DETECTION PORTION | ← ACOUSTIC WAVE A

IMAGER BODY PORTION

3

2

| LIGHT SOURCE DRIVING PORTION | 22

LIGHT TRIGGER SIGNAL

21

DETECTION SIGNAL

23

| CONTROL PORTION |

SAMPLING TRIGGER SIGNAL

| IMAGING PORTION |

| IMAGE DISPLAY PORTION | 24

**EUROPEAN SEARCH REPORT**

Application Number

EP 15 18 0843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2013/094170 A1 (FUJIFILM CORP [JP])<br>27 June 2013 (2013-06-27)<br>* abstract *<br>* paragraphs [0011], [0016], [0020] -<br>[0022], [0026], [0032], [0036],<br>[0043], [0053], [0055] - [0057],<br>[0061]; figure 1; table 1 * | 1,2,7-9,<br>11<br>6 | INV.<br>A61B5/00 |
| X | JP 2001 318082 A (YAMANAKA ICHIJI; NEC CORP) 16 November 2001 (2001-11-16)<br>* paragraphs [0005], [0008], [0020],<br>[0030] - [0042]; figures 1,5 * | 1,2,11,<br>14 | |
| X | GB 2 322 941 A (OPTEL INSTR LIMITED [GB])<br>9 September 1998 (1998-09-09)<br>* page 8, line 36 - page 9, line 1 *<br>* page 16, line 3 - line 7 *<br>* page 22, line 1 - line 5 *<br>* page 25, line 28 - line 36 * | 1,3,11,<br>15 | |
| X | US 2013/031982 A1 (SATO AKIRA [JP] ET AL)<br>7 February 2013 (2013-02-07)<br>* paragraphs [0027], [0030]; figure 1 * | 1,2,<br>10-12 | TECHNICAL FIELDS SEARCHED (IPC)<br>A61B<br>G01N<br>G05D |
| X | US 2010/037695 A1 (TSUJITA KAZUHIRO [JP] ET AL) 18 February 2010 (2010-02-18)<br>* paragraphs [0009], [0013], [0028],<br>[0035], [0039], [0041] - [0042]; figure 1 * | 1-5,11 | |
| X | WO 2013/158154 A1 (SENO MEDICAL INSTR INC [US]; CLINGMAN BRYAN [US]; ZALEV JASON [CA]) 24 October 2013 (2013-10-24)<br>* paragraphs [0112], [0148], [0152];<br>figures 1,14,15 * | 1,2,11,<br>13 | |

-/--

The present search report has been drawn up for all claims

| Place of search | Date of completion of the search | Examiner |
|---|---|---|
| Munich | 18 January 2016 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

# EUROPEAN SEARCH REPORT

Application Number

EP 15 18 0843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHUN TUNG YEN ET AL: "Theoretical Investigation on Semiconductor Lasers with Passive Waveguides", IEEE JOURNAL OF QUANTUM ELECTRONICS, IEEE SERVICE CENTER, PISCATAWAY, NJ, USA, vol. 32, no. 1, 1 January 1996 (1996-01-01), XP011051295, ISSN: 0018-9197 * abstract * | 6 | |
| A | KENDERIAN SHANT ET AL: "Narrow band laser-generated surface acoustic waves using a formed source in the ablative regime", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 113, no. 1, 1 January 2003 (2003-01-01), pages 261-266, XP012003228, ISSN: 0001-4966, DOI: 10.1121/1.1529664 * page 261, left-hand column, paragraph 1 * | 1 | |
| A | US 2011/275890 A1 (WANG LIHONG [US] ET AL) 10 November 2011 (2011-11-10) * paragraph [0052] * | 4,5 | TECHNICAL FIELDS SEARCHED (IPC) |

The present search report has been drawn up for all claims

| Place of search | Date of completion of the search | Examiner |
|---|---|---|
| Munich | 18 January 2016 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 18 0843

18-01-2016

| Patent document cited in search report | | Publication date | Patent family member(s) | | Publication date |
|---|---|---|---|---|---|
| WO 2013094170 | A1 | 27-06-2013 | JP | 2013128722 A | 04-07-2013 |
| | | | WO | 2013094170 A1 | 27-06-2013 |
| JP 2001318082 | A | 16-11-2001 | JP | 3607569 B2 | 05-01-2005 |
| | | | JP | 2001318082 A | 16-11-2001 |
| GB 2322941 | A | 09-09-1998 | GB | 2322941 A | 09-09-1998 |
| | | | GB | 2357844 A | 04-07-2001 |
| | | | GB | 2357845 A | 04-07-2001 |
| | | | GB | 2357846 A | 04-07-2001 |
| US 2013031982 | A1 | 07-02-2013 | JP | 5538855 B2 | 02-07-2014 |
| | | | JP | 2011120795 A | 23-06-2011 |
| | | | US | 2013031982 A1 | 07-02-2013 |
| | | | WO | 2011070985 A1 | 16-06-2011 |
| US 2010037695 | A1 | 18-02-2010 | JP | 5210087 B2 | 12-06-2013 |
| | | | JP | 2010042158 A | 25-02-2010 |
| | | | US | 2010037695 A1 | 18-02-2010 |
| WO 2013158154 | A1 | 24-10-2013 | NONE | | |
| US 2011275890 | A1 | 10-11-2011 | US | 2011275890 A1 | 10-11-2011 |
| | | | WO | 2010080991 A2 | 15-07-2010 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012196308 A

**Non-patent literature cited in the description**

- Medical Ultrasonic Equipment Handbook. Corona Publishing Co., Ltd, April 1985